# FASCICULE DE BREVET EUROPEEN

(11) **EP 2 539 311 B1**
(45) Date de publication et mention de la délivrance du brevet: **02.11.2016**
(21) Numéro de dépôt: 11714392.5
(22) Date de dépôt: 17.02.2011
(51) Int. Cl.: C08F 2/22, C08F 220/18, C09D 5/02, C08F 283/06, C08F 220/28, C09D 7/00

(54) **MONOMERE ASSOCIATIF A BASE D'ALCOOLS OXO, POLYMERE ACRYLIQUE CONTENANT CE MONOMERE, UTILISATION DUDIT POLYMERE COMME EPAISSISSANT DANS UNE FORMULATION AQUEUSE, FORMULATION OBTENUE**
ASSOZIATIVES MONOMER AUF DER BASIS VON OXOALKOHOLEN, ACRYLPOLYMER MIT DIESEM MONOMER, VERWENDUNG VON DIESEM POLYMER ALS VERDICKUNGSMITTEL IN EINER WÄSSRIGEN FORMULIERUNG UND ERHALTENE FORMULIERUNG
ASSOCIATIVE MONOMER BASED ON OXO ALCOHOLS, ACRYLIC POLYMER CONTAINING THIS MONOMER, USE OF SAID POLYMER AS THICKENER IN AN AQUEOUS FORMULATION, AND FORMULATION OBTAINED

(30) Priorité: 17.03.2010 US 314754 P; 26.02.2010 FR 1051366
(43) Date de publication de la demande: 02.01.2013
(73) Titulaire: COATEX, 69730 Genay (FR)
(72) Inventeur: SUAU, Jean-Marc, F-69480 Lucenay (FR); RUHLMANN, Denis, F-69730 Genay (FR)
(74) Mandataire: Balmefrezol, Ludovic Francis Pierre
(86) Numéro de dépôt international: PCT/IB2011/000327
(87) Numéro de publication internationale: WO 2011/104599

(56) Documents cités:
- EP-A1- 2 003 152
- WO-A1-2006/016035
- WO-A2-2006/130675
- US-A- 4 668 410
- DATABASE REGISTRY [Online] CHEMICAL ABSTRACTS SERVICE, COLUMBUS, OHIO, US; 16 November 1984 (1984-11-16), "Poly(oxy-1,2-ethanediyl),.alpha.-[4-[2-hy droxy-3-[(2-methyl-1-oxo-2-propenyl)oxy]pr opoxy]-1,4-dioxobutyl]-.omega.-[(5-methylh eptyl)oxy]-(9CI)", XP002652640, Database accession no. 52656-51-6 -& JP 48 033993 B 18 October 1973 (1973-10-18)

## Description

La présente invention concerne de nouveaux modificateurs de rhéologie de type HASE, dont le monomère associatif est fonctionnalisé par un groupe hydrophobe à base d'alcools oxo. Mis en oeuvre dans des formulations contenant de l'eau telles que des peintures aqueuses, ces produits conduisent à un phénomène d'augmentation de la viscosité sur une large amplitude de gradients de cisaillement, et tout particulièrement pour des gradients élevés : ils se comportent donc comme des épaississants efficaces, et constituent à ce titre une nouvelle gamme de produits qui vient enrichir la bibliothèque du formulateur de peintures.

De manière tout à fait avantageuse, ces nouveaux épaississants peuvent développer, lorsqu'ils sont introduits dans une formulation aqueuse, des profils rhéologiques bien plus newtoniens que leurs prédécesseurs de type HASE et ce, en préservant un niveau d'efficacité économiquement intéressant. Un tel résultat est obtenu par la mise en oeuvre des monomères associatifs particuliers précités. Cette propriété est même exacerbée dans une variante préférentielle qui consiste à ajuster dans un certain intervalle la dose d'agent de transfert de chaîne mis en oeuvre dans le procédé de fabrication desdits polymères.

Les monomères associatifs fonctionnalisés par des groupes hydrophobes à base d'alcools oxo constituent un objet de la présente invention. Il en va de même pour les polymères de type HASE incorporant de tels monomères, et pour leur procédé de synthèse. L'utilisation de ces derniers comme épaississants dans des formulations aqueuses et les formulations résultantes constituent les deux derniers objets de la présente invention.

Maîtriser la rhéologie d'une peinture, tant au stade de sa fabrication, que pendant son transport, son stockage ou sa mise en oeuvre reste aujourd'hui une priorité. La diversité des contraintes observées au niveau de chacune de ces étapes renvoie à une multiplicité de comportements rhéologiques différents. On peut néanmoins résumer le besoin de l'homme du métier à l'obtention d'un effet d'épaississement de ladite peinture, tant pour des raisons de stabilité au cours du temps, que pour une possible application sur une surface verticale, l'absence d'éclaboussure au moment de la mise en oeuvre, ou de coulure par la suite, etc... De fait, on a désigné les produits qui contribuent à cette régulation du comportement rhéologique sous le terme d'épaississants.

Historiquement, on a utilisé dès les années 1950 des gommes et des produits à base cellulosique, dont une des caractéristiques essentielles est leur poids moléculaire élevé. Ces composés présentent cependant un certain nombre d'inconvénients tels que leur instabilité au cours du temps (voir le document US 4 673 518), la nécessité d'en utiliser une quantité importante (voir le document EP 0 250 943 A1), ou leur coût de production, notamment au niveau du traitement des déchets (voir le document US 4 384 096).

Ont ensuite vu le jour les épaississants dits « associatifs » : ce sont des polymères hydrosolubles disposant de groupements hydrophobes insolubles. De telles macromolécules ont un caractère associant : une fois introduits dans l'eau, les groupements hydrophobes sont susceptibles de s'assembler sous forme d'agrégats micellaires. Ces agrégats sont reliés entre eux par les parties hydrophiles des polymères : il y a alors formation d'un réseau tridimensionnel qui provoque l'augmentation de la viscosité du milieu. Leur mécanisme de fonctionnement et leurs caractéristiques sont désormais bien connus et décrits par exemple dans les documents « Rheology modifiers for water-borne paints » (Surface Coatings Australia, 1985, pp. 6-10) et « Rheological modifiers for water-based paints : the most flexible tools for your formulations » (Eurocoat 97, UATCM, vol. 1, pp 423-442).

Parmi ces épaississants associatifs, on distingue la catégorie des HEUR (Uréthane oxyde d'Ethylène modifiés Hydrophobiquement ou Hydrophobically modified Ethylene oxyde URethane selon l'acronyme anglo-saxon approprié) et des HASE (Emulsions modifiées hydrophobiquement et épaississantes en milieu alcalin ou Hydrophobically modified Alkali-soluble Emulsions). Les premiers désignent des polymères résultant de la synthèse entre un composé du type polyalkylène glycol, un polyisocyanate, et un monomère associatif alkyle et/ou aryle constitué d'un groupe terminal hydrophobe. Les seconds désignent des polymères de l'acide (méth)acrylique, d'un ester de ces acides et d'un monomère associatif constitué d'une chaîne oxyalkylée terminée par un groupement hydrophobe.

Les HEUR sont à l'origine de nombreuses propriétés dans les peintures aqueuses, notamment fonction de la nature de leur monomère associatif. On pourra citer les demandes de brevet suivantes déposées par la société COATEX™ : EP 0 639 595 A1 qui propose des groupements hydrophobes ayant de 4 à 36 atomes de carbone pour augmenter la viscosité Brookfield™, WO 02 / 102868 A1 qui décrit l'utilisation de plurystyrylphénols avec plus de 40 atomes pour augmenter la viscosité quel que soit le gradient de cisaillement, et enfin EP 1 425 325 A1 qui révèle un monomère associatif constitué de di- et tristyrylphénol, permettant d'obtenir une excellente compatibilité pigmentaire et une viscosité élevée à bas et moyen gradient de cisaillement.

Il en va de même pour les HASE, produits pour lesquels on peut notamment mentionner les demandes de brevet suivantes déposées par COATEX™ : EP 0 577 526 A1 qui décrit une chaîne grasse avec des groupes linéaires ou ramifiés du type alkyle et/ou aryle ayant de 26 à 30 atomes de carbone, pour développer de hautes viscosités sous bas gradient de cisaillement, et EP 1 778 797 A1 qui décrit une chaîne terminale ramifiée comportant de 10 à 24 atomes de carbone, pour améliorer la compatibilité pigmentaire et augmenter la viscosité de manière générale.

En matière de rhéologie des peintures, s'il est intéressant de disposer d'une nouvelle famille de produits qui permet d'augmenter la viscosité sur une large gamme de gradient de cisaillement, il est aussi très utile de posséder des épaississants qui vont induire un profil rhéologique particulier, fonction du cahier des charges de l'applicateur ou du formulateur. A cet égard, on lit souvent que les peintures brillantes et satinées s'accommodent avantageusement d'un profil « newtonien », ce dernier permettant une amélioration de leur étalement et une réduction des projections pendant leur application.

Un profil « newtonien » signifie de manière théorique que la viscosité reste indépendante du gradient de cisaillement : un tel comportement est inaccessible dans la réalité. En pratique, l'homme du métier associe un tel profil à une faible évolution de la viscosité en fonction du taux de cisaillement (rhéogramme d'autant plus plat), par opposition à un profil pseudo-plastique caractérisé par une chute marquée de la viscosité en fonction de ce gradient (rhéogramme d'autant plus pentu). Concrètement, plus le rapport entre la viscosité Brookfield à 10 tours par minute en mPa.s et la viscosité cône-plan dite « ICI » (correspondant approximativement à un gradient de vitesse de 10 000 s⁻¹) est faible, plus on se rapproche d'un comportement newtonien.

Ceci étant, pour autant qu'on diminue le rapport indiqué ci-dessus, il est souhaitable de ne pas perdre en efficacité épaississante à haut gradient de cisaillement : ceci constitue une seconde exigence, qui peut se traduire par la recherche de manière concomitante d'une viscosité ICI la plus élevée possible.

Aujourd'hui, autant la technologie des HEUR que celle des HASE permet de formuler des épaississants associatifs, qui développent des profils newtoniens une fois introduits dans des formulations aqueuses. Ceci est notamment décrit dans le document « Disperse phase - thickeners interactions and their influence on Newtonian to non-Newtonian flow behavior » (Polymeric Materials, 1995), 73, 195-6).

Pour les HEUR, on pourra se reporter au document US 5 500 475, décrivant un épaississant de cette catégorie qui, une fois mis en oeuvre dans une peinture aqueuse, développe un profil newtonien tout en conduisant à un film sec ayant une brillance élevée, résistant à l'eau, à l'abrasion et à la contamination microbienne.

Cependant, les HEUR sont des espèces chimiques difficilement solubles dans l'eau et doivent être mis en solution en présence de solvants ou de tensio-actifs, dès lors que leur % en matière active excède environ 25 %, et pour autant qu'ils développent des profils newtoniens. Cette problématique est relatée dans le document EP 0 682 094 A1 : la solution proposée repose de manière triviale sur l'emploi de tensio-actifs. De plus, on notera que des épaississants de type HEUR, hautement concentrés et contenant des tensio-actifs, avaient été commercialisés par la société COATEX sous les noms COAPUR™ 5035 et COAPUR™ 6050, avant la date de priorité de cette demande, et dès 1993 pour les premiers d'entre eux.

Or, la mise en oeuvre de solvants et de tensio-actifs pour formuler l'épaississant de type HEUR n'est pas sans poser un certain nombre de problèmes. On rappelle d'une part que les solvants sont soumis à des législations de plus en plus draconiennes visant à restreindre et même proscrire leur utilisation dans les peintures. Quant aux tensio-actifs de la formulation épaississante, ils sont de nature à déstabiliser les peintures, par interaction avec les autres tensio-actifs contenues dans ces dernières. Par conséquent, la formulation de HEUR de type newtonien dans l'eau n'est uniquement possible qu'à condition de restreindre drastiquement la concentration en polyuréthane (de l'ordre de 20 % en poids), ce qui rend ces épaississants peu efficaces.

Parmi les HASE, les seuls qui s'avèrent véritablement efficaces pour développer des profils newtoniens sont des épaississants dont le monomère associatif contient des alkylphénols éthoxylés (APEs selon l'acronyme anglais correspondant). On en trouve une illustration dans le document EP 0 350 414 A1, qui enseigne l'emploi de nonylphénols éthoxylés pour la synthèse du monomère associatif d'un épaississant de type HASE, et démontre que le polymère résultant permet de développer des profils rhéologiques newtoniens une fois introduit dans une peinture aqueuse. Néanmoins, les alkylphénols sont aujourd'hui largement suspectés d'être cancérigènes et dangereux pour la reproduction ; encore tolérés dans l'industrie des peintures, ils n'en demeurent pas moins dans le collimateur des institutions législatives en la matière, notamment européennes.

Or, la Demanderesse est parvenue à mettre au point de nouveaux monomères associatifs, susceptibles de rentrer dans la composition de polymères de type HASE, apportant à l'homme du métier les avantages suivants :
- la possibilité de décliner, sur la base de ces nouveaux monomères associatifs, une nouvelle gamme d'épaississants de type HASE, capables d'épaissir une formulation aqueuse sur un large intervalle de gradient de cisaillement (et notamment sur la plage des gradients élevés), évitant ainsi le recours aux HEUR qui nécessitent solvants et/ou tensio-actifs dans leur formulation pour être efficaces ;
- en outre, ces nouveaux épaississants permettent de développer des profils newtoniens particulièrement marqués, notamment en terme de rapport (viscosité Brookfield à 10 tours par minute en mPa.s / viscosité ICI) et ce, tout en maintenant une viscosité ICI importante ;
- enfin, cette dernière propriété est exacerbée selon une variante particulière de l'invention, qui consiste à ajuster le taux d'agent de transfert de chaîne dans un certain intervalle, au cours du procédé de fabrication de ces épaississants.

Lesdits épaississants contiennent un monomère associatif de formule (I) :

R-(AO)ₘ-(BO)ₙ-R'

où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant caractérisé en ce qu'il est constitué d'au moins un groupement de formule (II) :

   CH₃-(CH₂)ₚ-CH(CH₂)ᵣCH₃-(CH₂)q-

   où p et q désignent des entiers dont un au moins est non nul, avec 5 < p + q < 13, r est un entier compris entre 0 et 5, préférentiellement égal à 0.

Une des clés de la présente invention repose sur le choix des composés de formule (II), en tant que groupe hydrophobe terminal du monomère associatif de formule (I). Ce dernier est fabriqué classiquement par éthoxylation d'un alcool, puis fonctionnalisation en vue de le rendre polymérisable. Dans le cadre de la présente invention, la Demanderesse a su remarquer que le choix particulier d'un alcool oxo permettait la synthèse de nouveaux monomères associatifs de formule (I), qui confèrent au final l'ensemble des propriétés énumérées précédemment.

La formule (III) de ces alcools oxo est la suivante :

CH₃-(CH₂)ₚ-CH(CH₂)ᵣCH₃-(CH₂)_{q}-OH

où p, q et r ont les significations déjà indiquées. Il s'agit de composés bien connus, obtenus de manière très simple par hydroformylation d'un alcène au moyen d'un gaz de synthèse présentant un rapport H₂/CO proche de 1. Cette réaction permet de transformer l'alcène en aldéhyde, lequel n'a plus qu'à être hydrogéné pour que l'alcool puisse être obtenu. On en trouve une illustration dans le document WO 2007 / 066036. Des exemples commerciaux de ces alcools sont les produits commercialisés par la société SASOL™ sous les marque Liai™, Isalchem™, Alchem™ et Safol™, ou par la société BASF™ sous la marque Lutensol™

Le fait d'indiquer que le groupement terminal R' du monomère de formule (I) est constitué d'au moins un groupement de formule (II) -et non pas qu'il est exclusivement constitué de ce groupement- relève du fait que l'alcool oxo de départ résulte de la réaction d'hydroformylation mentionnée ci-dessus, qui peut conduire aussi à la formation d'alcools linéaires. De plus, dans cette formule, la valeur indiquée pour p + q est celle relative à l'espèce majoritaire, les alcools oxo commerciaux étant en général des mélanges ou des coupes.

Si ces alcools ont déjà été utilisés en détergence (voir les documents EP 1 294 837 et US 4 280 919), ils n'ont encore jamais été mis en oeuvre pour la synthèse d'un monomère associatif. Enfin, les alcools oxo sont bien connus pour présenter une biodégradabilité élevée (voir le document US 4 280 919 précité), ce qui constitue un des autres avantages de la présente invention.

Aussi, un premier objet de l'invention consiste en un monomère de formule (I) :

R-(AO)ₘ-(BO)ₙ-R'

où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant caractérisé en ce qu'il est constitué d'au moins un groupement de formule (II) :

   CH₃-(CH₂)ₚ-CH(CH₂)ᵣCH₃-(CH₂)_{q}-

   où p et q désignent des entiers dont un au moins est non nul, avec 5 < p + q < 13, r est un entier compris entre 0 et 5, préférentiellement égal à 0.

Préférentiellement, ce monomère est caractérisé en ce que n = 0, AO désigne l'oxyde d'éthylène, et m est compris entre 20 et 40.

Un deuxième objet de la présente invention consiste en un polymère de type HASE, constitué :
a) d'acide (méth)acrylique,
b) d'au moins un ester de l'acide (méth)acrylique,
c) d'au moins un monomère ayant la formule (I) précitée.

La Demanderesse précise que la fabrication de ces copolymères, est parfaitement connue de l'homme du métier, qui pourra se reporter à l'enseignement des documents cités auparavant dans l'arrière plan technologique de la présente invention. Sur l'influence de la quantité d'agent de transfert de chaîne mise en oeuvre dans leur synthèse, qui fait aussi partie des objets revendiqués dans la présente Demande, toutes les précisions sont données ultérieurement.

Ce polymère est aussi caractérisé en ce qu'il est constitué, exprimé en % en poids de chacun de ses monomères :
a) de 20 % à 50 %, préférentiellement de 35 % à 45 % d'acide (méth)acrylique,
b) de 40 % à 70 %, préférentiellement de 45 % à 55 % d'au moins un ester de l'acide (méth)acrylique,
c) de 2 % à 20 %, préférentiellement de 3 % à 15 % d'au moins un monomère ayant la formule (I) précitée.

Dans une variante préférentielle, ce polymère est caractérisé en ce que, pour le monomère de formule (I), on a n = 0, AO désigne l'oxyde d'éthylène, et m est compris entre 20 et 40.

Un troisième objet de la présente invention consiste en un procédé de fabrication d'un polymère de type HASE, par mise en contact dans un milieu réactionnel entre :
a) de l'acide (méth)acrylique,
b) au moins un ester de l'acide (méth)acrylique,
c) au moins un monomère ayant la formule (I) précitée,
avec introduction éventuelle dans le milieu réactionnel, avant et/ou pendant et/ou après la mise en contact entre les constituant a), b) et c), d'au moins un agent de transfert de chaîne.

Dans une variante préférentielle, ledit procédé est caractérisé en ce que l'agent de transfert est mis en oeuvre et en ce que sa masse introduite dans le milieu réactionnel est comprise entre 1 500 ppm et 4 000 ppm par rapport au poids total des constituants a), b) et c). De manière tout à fait inattendue, on améliore à la fois le caractère newtonien du profil rhéologique induit par le polymère dans l'eau, tout en maintenant une efficacité épaississante importante à haut gradient de cisaillement.

Dans une variante préférentielle, le procédé est caractérisé en ce que, pour le monomère de formule (I), on a n = 0, AO désigne l'oxyde d'éthylène, et m est compris entre 20 et 40.

Ce résultat est contraire à l'enseignement général que l'introduction d'un agent de transfert de chaîne va obligatoirement diminuer l'efficacité épaississante du polymère (voir notamment page 7 du document EP 0 013 836 A1). De manière plus précise, on sait aussi qu'un excès d'agent de transfert (au moins 0,1 % -1 000 ppm- en poids par rapport au poids total des monomères engagés) conduit à une amélioration des profils newtoniens et de manière concomitante à une perte de pouvoir épaississant sur toute la gamme de (« Tailoring HASE Rheology through Polymer Design », JCT Research, vol. 2, n° 6, Avril 2005, pp. 423-433).

Ce procédé est aussi caractérisé en ce que le milieu réactionnel est constitué d'eau avec un autre solvant organique, préférentiellement uniquement constitué d'eau.

Ce procédé est aussi caractérisé en ce que l'agent de transfert de chaîne est choisi parmi le N-dodécyl mercaptan, le N-décyl mercaptan, l'octyl mercaptan, le 1,8-dimercapto-3,6-dioxaoctane (DMDO, n° CAS : 14970-87-7), l'acide thiolactique.

Un quatrième objet de la présente invention consiste en l'utilisation d'un polymère de type HASE tel qu'explicité auparavant, comme agent épaississant dans une formulation aqueuse.

Un cinquième et dernier objet de la présente invention consiste en une formulation aqueuse contenant un polymère de type HASE tel qu'explicité auparavant, dont une formulation de peinture aqueuse.

### EXEMPLES

### Exemple 1

Cet essai illustre la mise en oeuvre d'épaississants associatifs selon l'art antérieur (HASE) et selon l'invention, en vue d'épaissir un gel aqueux.
Pour chacun des essais n° 1 à 6, on introduit dans 215 grammes d'une solution aqueuse constituée de 40 grammes d'eau déionisée et 175 grammes d'un liant acrylique Neocryl XK 90 commercialisé par la société DSM™ une masse fixe égale à 10,7 grammes de l'épaississant à tester ainsi qu'environ 1 gramme d'une solution neutralisante d'hydroxyde d'ammonium à 28 %.

### Essai n° 1

Cet essai illustre l'art antérieur et met en oeuvre un épaississant associatif de type HASE commercialisé par la société COATEX™ sous le nom Rheotech™ 2100. Cet épaississant contient des alkylphénols.

### Essai n° 2

Cet essai illustre l'art antérieur et met en oeuvre un épaississant associatif de type HASE commercialisé par la société ROHM & HAAS™ sous le nom Acrysol™ RM5.

### Essai n° 3

Cet essai illustre l'invention et met en oeuvre un épaississant associatif de type HASE selon l'invention, qui est un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle
m = 30, n = 0, AO désigne l'oxyde d'éthylène, R désigne la fonction méthacrylate, R' désigne le groupement de formule (II) avec p + q = 10, r = 0.

Ce polymère a été obtenu par mise en contact dans un milieu réactionnel qui est l'eau, de l'ensemble des monomères précités et ce, en présence d'un agent de transfert de chaîne qui est le dodecyl mercaptan.

Concrètement, dans un réacteur de 1 litre on pèse 409 grammes d'eau bi permutée et 5,6 grammes de dodecyl sulfate de sodium. On chauffe en pied de cuve à 82°C ± 2°C.

Pendant ce temps, on prépare une pré émulsion, en pesant dans un bêcher :
- 116,8 grammes d'eau bi permutée,
- 1,96 gramme de dodecyl sulfate de sodium,
- 0,139 gramme de dodecyl mercaptan, soit 556 ppm de mercaptan par rapport à la masse de tous les monomères engagés
- 88,84 grammes d'acide méthacrylique,
- 131,1 grammes d'acrylate d'éthyle,
- 30,0 grammes de macromonomère

On pèse ensuite 0,8 gramme de persulfate d'ammonium dilué dans 6 grammes d'eau bi permutée pour le premier catalyseur, et 0,08 gramme de métabisulfite de sodium dilués dans 4 grammes d'eau bi permutée pour le second catalyseur. Lorsque le pied de cuve est à température, on ajoute les 2 catalyseurs et on effectue la polymérisation pendant 2 heures à 84°C ± 2°C, avec ajout en parallèle de la pré émulsion. On rince la pompe avec 20 grammes d'eau bi permutée et on cuit 1 heure à 84°C ± 2°C. On refroidit enfin à température ambiante et on filtre.

### Essai n° 4

Cet essai illustre l'invention et met en oeuvre un épaississant associatif de type HASE selon l'invention, qui est un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle
m = 25, n = 0, AO désigne l'oxyde d'éthylène, R désigne la fonction méthacrylate, R' désigne le groupement de formule (II) avec p + q = 10, r = 0.
Ce monomère a été obtenu par éthoxylation du produit commercial Lial 123, puis rendu polymérisable par fonctionnalisation à l'aide d'un groupement méthacrylate. L'éthoxylation est réalisée par des méthodes bien connues de l'homme du métier. Il en est de même pour la fonctionnalisation.
Ce polymère a été obtenu par mise en contact dans un milieu réactionnel qui est l'eau, de l'ensemble des monomères précités et ce, en présence de 560 ppm d'un agent de transfert de chaîne qui est le dodecyl mercaptan.
Sa synthèse a été réalisée comme indiqué au niveau de l'essai n° 3.

### Essai n° 5

Cet essai illustre l'invention et met en oeuvre un épaississant associatif de type HASE selon l'invention, qui est un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle
m = 25, n = 0, AO désigne l'oxyde d'éthylène, R désigne la fonction méthacrylate, R' désigne le groupement de formule (II) avec p + q = 10, r = 0.
Ce monomère a été obtenu par éthoxylation du produit commercial Lial 123, puis rendu polymérisable par fonctionnalisation à l'aide d'un groupement méthacrylate. L'éthoxylation est réalisée par des méthodes bien connues de l'homme du métier. Il en est de même pour la fonctionnalisation.
Ce polymère a été obtenu par mise en contact dans un milieu réactionnel qui est l'eau, de l'ensemble des monomères précités et ce, en présence de 560 ppm d'un agent de transfert de chaîne qui est le dodecyl mercaptan.
Sa synthèse a été réalisée comme indiqué au niveau de l'essai n° 3.

### Essai n° 6

Cet essai illustre l'invention et met en oeuvre un épaississant associatif de type HASE selon l'invention, qui est un polymère constitué de, en % en poids de chacun de ses monomères :
a) 34,0 % d'acide méthacrylique,
b) 51,0 % d'acrylate d'éthyle,
c) 15,0 % d'un monomère de formule (I) dans laquelle
m = 25, n = 0, AO désigne l'oxyde d'éthylène, R désigne la fonction méthacrylate, R' désigne le groupement de formule (II) avec p + q = 10, r = 0.
Ce monomère a été obtenu par éthoxylation du produit commercial Lial 123, puis rendu polymérisable par fonctionnalisation à l'aide d'un groupement méthacrylate. L'éthoxylation est réalisée par des méthodes bien connues de l'homme du métier. Il en est de même pour la fonctionnalisation.
Ce polymère a été obtenu par mise en contact dans un milieu réactionnel qui est l'eau, de l'ensemble des monomères précités et ce, en présence de 560 ppm d'un agent de transfert de chaîne qui est le dodecyl mercaptan.
Sa synthèse a été réalisée comme indiqué au niveau de l'essai n° 3.

Les viscosités Brookfield™ à 10 et 100 tours par minute (Brook 10T, Brook 100T en mPa.s), Stormer™ (en KU) et ICI™, de même que le rapport entre la viscosité Brookfield™ à 10 tours / minute et la viscosité ICI™ ont été reportés dans le tableau 1.

**Tableau 1**

| **Essai n°** | **1** | **2** | **3** | **4** | **5** | **6** |
|---|---|---|---|---|---|---|
| **Art Antérieur / Invention** | **AA** | **AA** | **IN** | **IN** | **IN** | **IN** |
| ICI | 2,3 | 1,1 | 3,8 | 3 | 2,3 | 3,8 |
| Brook.10T | 4120 | 3560 | 7800 | 3560 | 3320 | 6560 |
| Brook.100T | 2940 | 1300 | 4900 | 2430 | 1840 | 4300 |
| Stormer | 113 | 83 | 134 | 108 | 98 | 129 |
| Brook.10T/ICI | 1791 | 3236 | 2053 | 1187 | 1443 | 1726 |

Ces résultats démontrent que les polymères HASE de l'invention permettent d'obtenir un profil newtonien plus marqué qu'avec le polymère HASE de l'art antérieur référencé RM 5 et ce, avec une efficacité épaississante bien plus importante à haut gradient de cisaillement.

Au niveau du caractère newtonien, la performance du produit Rheotech™ 2100 contenant des alkyl phénols est au moins égalée, et elle est largement améliorée dans le cadre de la viscosité ICI.

### Exemple 2

Cet essai illustre la mise en oeuvre d'épaississants associatifs selon l'invention, en vue d'épaissir un gel aqueux. Il illustre notamment l'influence de la quantité d'agent de transfert de chaine mise en oeuvre pendant la synthèse dudit épaississant.
Pour chacun des essais n° 7 à 12, on introduit dans 215 grammes d'une solution aqueuse constituée de 40 grammes d'eau déionisée et 175 grammes d'un liant acrylique Neocryl XK 90 commercialisé par la société DSM™ une masse fixe égale à 10,7 grammes de l'épaississant à tester ainsi qu'environ 1 gramme d'une solution neutralisante d'hydroxyde d'ammonium à 28 %.

### Essai n° 7

Cet essai illustre l'art antérieur et met en oeuvre un épaississant associatif de type HEUR commercialisé par la société COATEX™ sous le nom Coapur™ 2025.

### Essai n° 8

Cet essai illustre l'invention et met en oeuvre un épaississant associatif de type HASE selon l'invention, qui est un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle
m = 25, n = 0, AO désigne l'oxyde d'éthylène, R désigne la fonction méthacrylate, R' désigne le groupement de formule (II) avec p + = 10, r = 0.

Ce monomère a été obtenu par éthoxylation du produit commercial Lial™ 123, puis rendu polymérisable par fonctionnalisation à l'aide d'un groupement méthacrylate. L'éthoxylation est réalisée par des méthodes bien connues de l'homme du métier. Il en est de même pour la fonctionnalisation.
Ce polymère a été obtenu par mise en contact dans un milieu réactionnel qui est l'eau, de l'ensemble des monomères précités et ce, en présence de 1392 ppm d'un agent de transfert de chaîne qui est le dodecyl mercaptan.
Sa synthèse a été réalisée comme indiqué au niveau de l'essai n° 3.

### Essai n° 9

Cet essai illustre l'invention et met en oeuvre un épaississant associatif de type HASE selon l'invention, qui est le même polymère que celui mis en oeuvre dans l'essai n° 8.
Ce polymère a été obtenu par mise en contact dans un milieu réactionnel qui est l'eau, de l'ensemble des monomères précités et ce, en présence de 1944 ppm d'un agent de transfert de chaîne qui est le dodecyl mercaptan.
Sa synthèse a été réalisée comme indiqué au niveau de l'essai n° 3.

### Essai n° 10

Cet essai illustre l'invention et met en oeuvre un épaississant associatif de type HASE selon l'invention, qui est le même polymère que celui mis en oeuvre dans l'essai n° 8.
Ce polymère a été obtenu par mise en contact dans un milieu réactionnel qui est l'eau, de l'ensemble des monomères précités et ce, en présence de 2840 ppm d'un agent de transfert de chaîne qui est le dodecyl mercaptan.
Sa synthèse a été réalisée comme indiqué au niveau de l'essai n° 3.

### Essai n° 11

Cet essai illustre l'invention et met en oeuvre un épaississant associatif de type HASE selon l'invention, qui est le même polymère que celui mis en oeuvre dans l'essai n° 8.
Ce polymère a été obtenu par mise en contact dans un milieu réactionnel qui est l'eau, de l'ensemble des monomères précités et ce, en présence de 4160 ppm d'un agent de transfert de chaîne qui est le dodecyl mercaptan.
Sa synthèse a été réalisée comme indiqué au niveau de l'essai n° 3.

### Essai n° 12

Cet essai illustre l'invention et met en oeuvre un épaississant associatif de type HASE selon l'invention, qui est le même polymère que celui mis en oeuvre dans l'essai n° 8.
Ce polymère a été obtenu par mise en contact dans un milieu réactionnel qui est l'eau, de l'ensemble des monomères précités et ce, en présence de 5600 ppm d'un agent de transfert de chaîne qui est le dodecyl mercaptan.
Sa synthèse a été réalisée comme indiqué au niveau de l'essai n° 3.

Les viscosités Brookfield™ à 10 et 100 tours par minute, Stormer™ et ICI™, de même que le rapport entre la viscosité Brookfield™ à 10 tours par minute et la viscosité ICI™ ont été reportés dans le tableau 2.

**Tableau 2**

| **Essai n°** | **7** | **8** | **9** | **10** | **11** | **12** |
|---|---|---|---|---|---|---|
| **Art Antérieur/Invention** | **AA** | **IN** | **IN** | **IN** | **IN** | **IN** |
| ICI | 2,2 | 3,3 | 3,2 | 2,9 | 2,5 | 2,2 |
| Brook10T (mPa.s) | 1520 | 3440 | 2120 | 1920 | 1720 | 1240 |
| Brook100T (mPa.s) | 1184 | 2610 | 1840 | 1630 | 1480 | 1130 |
| Stormer (KU) | 83 | 111 | 101 | 97 | 94 | 86 |
| Brook10T/ICI | 690 | 1042 | 663 | 662 | 688 | 564 |

Outre le fait que ce tableau continue à démontrer la capacité des polymères selon l'invention à épaissir un gel aqueux sur une large gamme de gradients de cisaillement, il illustre aussi l'influence du taux d'agent de transfert de chaîne sur la rhéologie de ce gel.

La valeur du rapport viscosité Brookfield à 10 tours par minute / viscosité ICI est largement diminuée lorsque ce taux augmente : on se rapproche d'un comportement très newtonien.
On obtient un compromis optimal entre ce rapport et le maintien de la viscosité ICI à un niveau élevé, pour les essais 9 et 10 qui illustrent la variante préférentielle de l'invention.

Selon cette variante, on améliore les résultats de l'épaississant HEUR de l'art antérieur au niveau du comportement newtonien, mais surtout de la performance épaississante à haut gradient de cisaillement.

### Exemple 3

### Essai n° 13

Cet essai illustre l'invention, ne met pas en oeuvre d'agent de transfert de chaîne, et concerne une émulsion aqueuse d'un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle m = 30, n = 0, AO désigne l'oxyde d'éthylène, R désigne la fonction méthacrylate, R' désigne le groupement de formule (II) avec p + q = 9, r = 0 (désigné sous le terme oxo C₁₂OE₃₀).

Ce polymère a été obtenu par mise en contact dans un milieu réactionnel qui est l'eau, de l'ensemble des monomères précités. Sa synthèse correspond à celle décrite dans les essais n° 14 à 19, mais sans mise en oeuvre d'agent de transfert de chaîne.

### Essai n° 14 à 19

Ces essais illustrent l'invention, et mettent en oeuvre une dose croissante d'un agent de transfert de chaîne qui est le dodecyl mercaptan (variante préférée du procédé de fabrication selon la présente invention), et concernent des émulsions aqueuses d'un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle m = 30, n = 0, AO désigne l'oxyde d'éthylène, R désigne la fonction méthacrylate, R' désigne le groupement de formule (II) avec p + q = 9, r = 0 (désigné sous le terme oxo C₁₂OE₃₀).

Ce polymère a été obtenu par mise en contact dans un milieu réactionnel qui est l'eau, de l'ensemble des monomères précités et ce, en présence d'une certaine quantité d'agent de transfert de chaîne qui est le dodecyl mercaptan.

Concrètement, pour la synthèse du polymère en présence de 560 ppm de mercaptan (essai n° 14), on commence par peser dans un réacteur de 1 litre 409 grammes d'eau bi permutée et 5,6 grammes de dodecyl sulfate de sodium. On chauffe en pied de cuve à 82°C ± 2°C.

Pendant ce temps, on prépare une pré émulsion, en pesant dans un bécher :
- 116,8 grammes d'eau bi permutée,
- 1,96 gramme de dodecyl sulfate de sodium,
- 0,139 gramme de dodecyl mercaptan, soit 556 ppm de mercaptan par rapport à la masse de tous les monomères engagés,
- 88,84 grammes d'acide méthacrylique,
- 131,1 grammes d'acrylate d'éthyle,
- 30,0 grammes de macromonomère.

On pèse ensuite 0,8 gramme de persulfate d'ammonium dilué dans 6 grammes d'eau bi permutée pour le premier catalyseur, et 0,08 gramme de métabisulfite de sodium dilué dans 4 grammes d'eau bi permutée pour le second catalyseur. Lorsque le pied de cuve est à température, on ajoute les 2 catalyseurs et on effectue la polymérisation pendant 2 heures à 84°C ± 2°C, avec ajout en parallèle de la pré émulsion. On rince la pompe avec 20 grammes d'eau bi permutée et on cuit 1 heure à 84°C ± 2°C. On refroidit enfin à température ambiante et on filtre.
Les synthèses correspondant aux essais n° 15 à 19 sont réalisées de manière similaire, en ajustant la dose de mercaptan.

### Observations

On constate que seuls les essais mettant à la fois en oeuvre le monomère particulier à base d'alcools oxo et l'agent de transfert de chaîne, conduisent à des valeurs de viscosité Brookfield™ faibles, même à un extrait sec de 25 % (tableau 4).
De plus, chacune de ces émulsions totalement préneutralisée, permet d'épaissir efficacement la solution de liant acrylique (tableau 5). Les meilleurs résultats sont notamment obtenus selon les émulsions des essais n° 17 et 18 qui, tout en ayant un viscosité Brookfield™ à 10 tours par minute inférieure à 20 000 mPa.s pour un extrait sec cependant égal à 25 %, conduisent néanmoins à un épaississement important, quel que soit le gradient de cisaillement.
On dispose donc d'émulsions préneutralisées de type HASE, stables et manipulables pour un extrait sec ayant un intérêt commercial, dénuées de nonyl phénols, et pouvant épaissir avantageusement un latex en solution aqueuse.

### Essai n° 20

Cet essai illustre l'invention. Il met notamment en oeuvre un monomère associatif différent de celui utilisé précédemment (lien méthacryl-uréthanne).

Il met en oeuvre une certaine quantité d'un agent de transfert de chaîne qui est le dodecyl mercaptan, et concerne une émulsion aqueuse d'un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle m = 30, n = 0, AO désigne l'oxyde d'éthylène, R désigne la fonction méthacryl-uréthanne, R' désigne le groupement de formule (II) avec p + q = 9, r = 0 (désigné sous le terme oxo C₁₂OE₃₀).

Ce polymère a été obtenu par mise en contact dans un milieu réactionnel qui est l'eau, de l'ensemble des monomères précités, selon la même procédure que décrite précédemment.

### Essais n° 21 et 22

Cet essai illustre l'invention. Il met notamment en oeuvre un monomère associatif différent de celui utilisé dans les essais précédents (similaire à celui utilisé dans les essais n° 14 à 19, mais oxyéthylé 25 fois, le % du monomère associatif étant ici de 10 et 15 %).

Ils mettent en oeuvre une certaine quantité d'un agent de transfert de chaîne qui est le dodecyl mercaptan, et concerne une émulsion aqueuse d'un polymère constitué de, en % en poids de chacun de ses monomères :
a) 33,5 % et 37,5 % d'acide méthacrylique (essais 9 et 10),
b) 52,5 % d'acrylate d'éthyle,
c) 10,0 % et 15,0 % d'un monomère de formule (I) dans laquelle (essais n° 21 et 22) m = 25, n = 0, AO désigne l'oxyde d'éthylène, R désigne la fonction méthacrylate, R' désigne le groupement de formule (II) avec p + q = 9, r = 0 (désigné sous le terme oxo C₁₂OE₂₅).

Ce polymère a été obtenu par mise en contact dans un milieu réactionnel qui est l'eau, de l'ensemble des monomères précités, selon la même procédure que décrite précédemment.

### Observations

Les résultats selon les essais 20 à 22 corroborent ceux obtenus selon les tests n° 14 à 19. On observe des valeurs de viscosité Brookfield™ faibles.
On dispose donc d'émulsions préneutralisées de type HASE, stables et manipulables pour un extrait sec ayant un intérêt commercial, dénuées de nonyl phénols, et pouvant épaissir avantageusement un latex en solution aqueuse.

### Essais n° 23 à 27

Ces essais illustrent des domaines hors invention. Ils mettent notamment en oeuvre un monomère associatif différent de celui utilisé dans les essais précédents, et exempt de nonylphénols. Ils mettent (essais 25 à 27) ou non (essais 23 et 24) en oeuvre 2 110 ppm d'un agent de transfert de chaîne qui est le dodecyl mercaptan.

Ils concernent une émulsion aqueuse d'un polymère constitué de, en % en poids de chacun de ses monomères :
a) 35,5 % d'acide méthacrylique,
b) 52,5 % d'acrylate d'éthyle,
c) 12,5 % d'un monomère qui est :
   un monomère de formule (I) dans laquelle m = 36, n = 0, AO désigne l'oxyde d'éthylène, R désigne la fonction méthacrylate, R' désigne le 2-hexyl 1-dodécanyle pour l'essai n° 23 (désigné sous le terme iso C₂₀OE₃₆) ;
   un monomère de formule (I) dans laquelle m = 25, n = 0, AO désigne l'oxyde d'éthylène, R désigne la fonction méthacrylate, R' désigne un groupe alkyle linéaire ayant 12 atomes de carbone pour l'essai n° 24 (désigné sous le terme C₁₂OE₂₅) ;
   le même monomère que celui selon l'essai n° 23 pour l'essai n° 25 ;
   un monomère de formule (I) dans laquelle m = 23, n = 0, AO désigne l'oxyde d'éthylène, R désigne la fonction méthacrylate, R' désigne un groupe alkyle linéaire ayant 12 atomes de carbone pour l'essai n° 26 ;
   le même monomère que celui selon l'essai n° 26 pour l'essai n° 27 ; cet essai particulier met en oeuvre 5 600 ppm de mercaptan.

Ces polymères ont été obtenus par mise en contact dans un milieu réactionnel qui est l'eau, de l'ensemble des monomères précités, selon la même procédure que décrite précédemment.

### Observations

On constate selon les essais n° 23 à 27 que le choix d'un monomère hors invention, ou le choix de ne pas utiliser d'agent de transfert de chaîne, conduit à des viscosités Brookfield™ à 10 tours par minute qui s'élèvent très rapidement, en fonction de l'extrait sec. Aucune de ces émulsions ne se prête donc à une pré neutralisation, sous peine d'être trop visqueuse.
Par comparaison entre les essais n° 19 et 27 qui mettent en oeuvre la plus grande quantité d'agent de transfert de chaîne (5 600 ppm), on constate les biens meilleurs résultats obtenus avec l'émulsion selon l'invention en termes de rhéologie en l'état.
Les essais n° 23 à 27 ne conduisant pas à des émulsions préneutralisées et manipulables en l'état à un extrait sec de 20 %, elles n'ont pas pu être testées sur le liant acrylique.

### Essai n° 28

Cet essai illustre un domaine hors invention et met en oeuvre une émulsion d'un polymère HASE commercialisée par la société COATEX™ sous le nom Rheotech™ 2100, qui contient des nonylphénols.

### Observations

On observe des valeurs de viscosité Brookfield™ à 10 tours par minute très faibles, même à 25 % d'extrait sec, ainsi qu'un pouvoir épaississant marqué sur le liant latex, quel que soit le gradient de cisaillement.
Néanmoins, cette émulsion contient des nonylphénols.

### Essais n° 29 et 30

Ces essais illustrent un domaine hors invention et mettent en oeuvre une émulsion d'un polymère HASE commercialisée sous les noms Acrysol™ TT 615 et Acrysol™ TT 935 par la société ROHM & HAAS™, qui ne contiennent pas le monomère particulier de la présente invention.

### Observations

Aucune de ces émulsions ne donne satisfaction en terme de viscosité en l'état, les valeurs de la viscosité Brookfield™ à 10 tours par minute étant extrêmement élevées, même à un extrait sec de 10 %. Elles n'ont pas été testées sur le liant acrylique.

**Tableau 3**

| **Essai n°** | **IN/HI** | **Monomère associatif** | **Dose agent de transfert (ppm / masse totale des monomères)** |
|---|---|---|---|
| **13** | **IN** | oxo C₁₂OE₃₀ | 0 |
| **14** | **IN** | oxo C₁₂OE₃₀ | 560 |
| **15** | **IN** | oxo C₁₂OE₃₀ | 1 390 |
| **16** | **IN** | oxo C₁₂OE₃₀ | 1 800 |
| **17** | **IN** | oxo C₁₂O₃₀ | 2840 |
| **18** | **IN** | oxo C₁₂O₃₀ | 4150 |
| **19** | **IN** | oxo C₁₂OE₃₀ | 5600 |
| **20** | **IN** | maeg TDI oxo C₁₂OE₃₀ | 1 390 |
| **21** | **IN** | oxo C₁₂OE₂₅ (10% monomère) | 2840 |
| **22** | **IN** | oxo C₁₂OE₂₅ (15 % monomère) | 2840 |
| **23** | **HI** | iso C₂₀OE₃₆ | 0 |
| **24** | **HI** | C₂₂OE₂₅ | 0 |
| **25** | **HI** | iso C₁₆OE₂₅ | 2 110 |
| **26** | **HI** | C₁₂OE₂₃ | 2110 |
| **27** | **HI** | C₁₂OE₂₃ | 5600 |

| | | | |
|---|---|---|---|
| IN = invention HI = hors invention | | | |

**Tableau 4**

| **Essai n°** | **IN/HI** | **Bk10 10%** | **Bk10 15%** | **Bk10 20%** | **Bk10 25 %** |
|---|---|---|---|---|---|
| **13** | **IN** | 20500 | >10⁵ | >10⁵ | Non mesurable |
| **14** | **IN** | 20500 | 24 000 | 46 000 | 120000 |
| **15** | **IN** | 18 000 | 22 000 | 36 800 | 80 000 |
| **16** | **IN** | 9000 | Non mesuré | 21 000 | 42000 |
| **17** | **IN** | 3 500 | 8 500 | 12 400 | 17 400 |
| **18** | **IN** | Non mesuré | 5 500 | 10 500 | 14200 |
| **19** | **IN** | Non mesuré | 3 500 | 8 300 | 10700 |
| **20** | **IN** | 7 000 | 18 400 | 32 400 | Non mesuré |
| **21** | **IN** | Non mesuré | | 11 200 | |
| **22** | **IN** | | | 13 800 | |
| **23** | **HI** | >10⁵ | Non mesurable | | |
| **24** | **HI** | >10⁵ | | | |
| **25** | **HI** | >10⁵ | | | |
| **26** | **HI** | 52 000 | | | |
| **27** | **HI** | 11 000 | 22 600 | 52 000 | >10⁵ |
| **28** | **HI** | 500 | Non mesuré | 2600 | 5 700 |
| **29** | **HI** | >10⁵ | Non mesurable | | |
| **30** | **HI** | >10⁵ | | | |

**Tableau 5**

| **Essai n°** | **IN/HI** | **Bk10** | **Bk100** | **Stormer** | **ICI** |
|---|---|---|---|---|---|
| **14** | **IN** | 5480 | 2540 | 102 | 2,4 |
| **15** | **IN** | 2320 | 1 360 | 87 | 2 |
| **16** | **IN** | 1 360 | 900 | 77 | 2 |
| **17** | **IN** | 1 320 | 840 | 75 | 1,8 |
| **18** | **IN** | 880 | 610 | 68 | 1,5 |
| **19** | **IN** | 760 | 530 | 66 | 1,3 |

A la lumière de ce dernier tableau, on constate que les émulsions selon l'invention permettent au formulateur d'obtenir une palette de comportements rhéologiques très variés.

### Exemple 4

Cet essai illustre la mise en oeuvre d'épaississants associatifs selon l'art antérieur (HASE) et selon l'invention, en vue d'épaissir un gel aqueux.
Pour chacun des essais n° 31 à 33, on introduit dans 215 grammes d'une solution aqueuse constituée de 40 grammes d'eau déionisée et 175 grammes d'un liant acrylique Neocryl XK 90 commercialisé par la société DSM™ une masse fixe égale à 10,7 grammes de l'épaississant à tester ainsi qu'environ 1 gramme d'une solution neutralisante d'hydroxyde d'ammonium à 28 %. Ce protocole est identique à celui décrit pour l'exemple n° 1.

### Essai n° 31 à 33

Ces essais illustrent l'invention, et mettent en oeuvre une dose croissante d'un agent de transfert de chaîne qui est le dodecyl mercaptan (variante préférée du procédé de fabrication selon la présente invention), et concernent des émulsions aqueuses d'un polymère constitué de, en % en poids de chacun de ses monomères :
a) 35,5 % d'acide méthacrylique,
b) 52,5 % d'acrylate d'éthyle,
c) 12,0 % d'un monomère de formule (I) dans laquelle AO désigne l'oxyde d'éthylène et BO l'oxyde de propylène, R désigne la fonction méthacrylate, R' désigne le groupement de formule (II) avec p + q = 9, r = 0 (désigné sous le terme oxo C₁₂OE₃₀), avec
   m = 50, n = 0 pour l'essai n° 31
   m = 40, n = 10 pour l'essai n° 32
   m = 15, n = 0 pour l'essai n° 33

Ces polymères ont été obtenus par mise en contact dans un milieu réactionnel qui est l'eau, de l'ensemble des monomères précités et ce, en présence de 560 ppm de dodecyl mercaptan. Par rapport à l'essai n° 14, on fait donc ici varier le nombre de motifs d'oxyde d'éthylène et de propylène.

Les viscosités Brookfield™ à 10 et 100 tours par minute (Brook 10T, Brook 100T en mPa.s), Stormer™ (en KU) et ICI™, de même que le rapport entre la viscosité Brookfield™ à 10 tours / minute et la viscosité ICI™ ont été reportés dans le tableau 6.

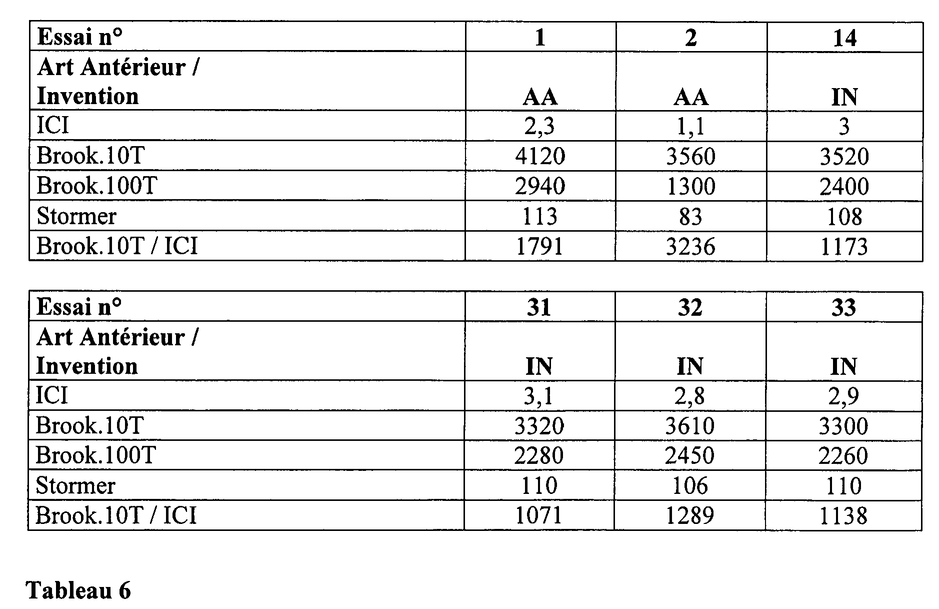

Ces résultats démontrent que les polymères HASE de l'invention permettent d'obtenir un profil newtonien plus marqué qu'avec le polymère HASE de l'art antérieur référencé RM 5 et ce, avec une efficacité épaississante bien plus importante à haut gradient de cisaillement.
Au niveau du caractère newtonien, la performance du produit Rheotech™ 2100 contenant des alkyl phénols est au moins égalée, et elle est largement améliorée dans le cadre de la viscosité ICI.

### Exemple 5

Cet essai illustre la mise en oeuvre d'épaississants associatifs selon l'art antérieur (HASE) et selon l'invention, en vue d'épaissir un gel aqueux.
Pour chacun des essais n° 34 à 36, on introduit dans 215 grammes d'une solution aqueuse constituée de 40 grammes d'eau déionisée et 175 grammes d'un liant acrylique Neocryl XK 90 commercialisé par la société DSM™ une masse fixe égale à 10,7 grammes de l'épaississant à tester ainsi qu'environ 1 gramme d'une solution neutralisante d'hydroxyde d'ammonium à 28 %. Ce protocole est identique à celui décrit pour l'exemple n° 1.

### Essai n° 34 à 36

Ces essais illustrent l'invention, et mettent en oeuvre une dose croissante d'un agent de transfert de chaîne qui est le dodecyl mercaptan (variante préférée du procédé de fabrication selon la présente invention), et concernent des émulsions aqueuses d'un polymère constitué de, en % en poids de chacun de ses monomères :
a) 38 %, 36 % et 33 % d'acide méthacrylique, respectivement pour les essais n° 34, 35 et 36
b) 57 %, 56 %, 54 %, % d'acrylate d'éthyle, respectivement pour les essais n° 34, 35 et 36
c) 5 %, 8 %, 15 % respectivement pour les essais n° 34, 35 et 36 d'un monomère de formule (I) dans laquelle AO désigne l'oxyde d'éthylène, m = 30, n = 0, R désigne la fonction méthacrylate, R' désigne le groupement de formule (II) avec p + q = 9, r = 0 (désigné sous le terme oxo C₁₂OE₃₀).

Ces polymères ont été obtenus par mise en contact dans un milieu réactionnel qui est l'eau, de l'ensemble des monomères précités et ce, en présence de 560 ppm de dodecyl mercaptan. Par rapport à l'essai n° 14, on fait donc ici varier les ratios monomériques.

Les viscosités Brookfïeld™ à 10 et 100 tours par minute (Brook 10T, Brook 100T en mPa.s), Stormer™ (en KU) et ICI™, de même que le rapport entre la viscosité Brookfïeld™ à 10 tours / minute et la viscosité ICI™ ont été reportés dans le tableau 7.

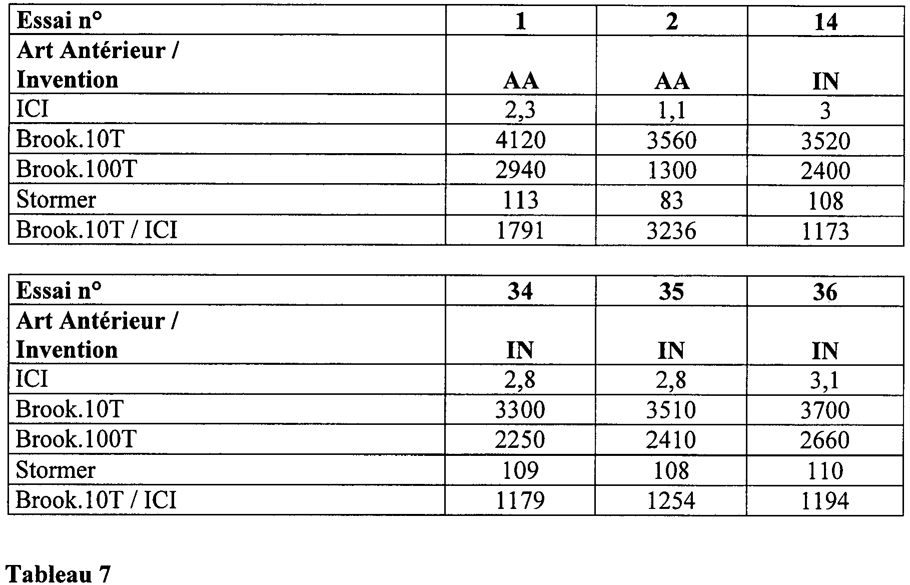

Ces résultats démontrent que les polymères HASE de l'invention permettent d'obtenir un profil newtonien plus marqué qu'avec le polymère HASE de l'art antérieur référencé RM 5 et ce, avec une efficacité épaississante bien plus importante à haut gradient de cisaillement.
Au niveau du caractère newtonien, la performance du produit Rheotech™ 2100 contenant des alkyl phénols est au moins égalée, et elle est largement améliorée dans le cadre de la viscosité ICI.

## Revendications

1. Monomère de formule (I) :
R-(AO)ₘ-(BO)ₙ-R'
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est constitué d'au moins un groupement de formule (II) :
CH₃-(CH₂)ₚ-CH(CH₂)ᵣCH₃-(CH₂)_{q}-
où p et q désignent des entiers dont un au moins est non nul, avec 5 < p + q < 13, r est un entier égal à 0.

2. Monomère selon la revendication 1, **caractérisé en ce que** n = 0, AO désigne l'oxyde d'éthylène, et m est compris entre 20 et 40.

3. Polymère de type HASE, constitué :
a) d'acide (méth)acrylique,
b) d'au moins un ester de l'acide (méth)acrylique,
c) d'au moins un monomère de formule (I),
R-(AO)ₘ-(BO)ₙ-R'
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est constitué d'au moins un groupement de formule (II) :
CH₃-(CH₂)ₚ-CH(CH₂)ᵣCH₃-(CH₂)_{q}-
où p et q désignent des entiers dont un au moins est non nul, avec 5<p+q<13, r est un entier égal à 0.

4. Polymère selon la revendication 3, **caractérisé en ce qu'**il est constitué, exprimé en % en poids de chacun de ses monomères :
a) de 20 % à 50 %, préférentiellement de 35 % à 45 % d'acide (méth)acrylique,
b) de 40 % à 70 %, préférentiellement de 45 % à 55 % d'au moins un ester de l'acide (méth)acrylique,
c) de 2 % à 20 %, préférentiellement de 3 % à 15 % d'au moins un monomère de formule (I).

5. Polymère selon l'une des revendications 3 ou 4, **caractérisé en ce que**, pour le monomère de formule (I), on a n = 0, AO désigne l'oxyde d'éthylène, et m est compris entre 20 et 40.

6. Procédé de fabrication d'un polymère de type HASE, par mise en contact dans un milieu réactionnel entre :
a) de l'acide (méth)acrylique,
b) au moins un ester de l'acide (méth)acrylique,
c) au moins un monomère de formule (I),
R-(AO)ₘ-(BO)ₙ-R'
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est constitué d'au moins un groupement de formule (II) :
CH₃-(CH₂)ₚ-CH(CH₂)ᵣCH₃-(CH₂)_{q}- (II)
où p et q désignent des entiers dont un au moins est non nul, avec 5 < p + q < 13, r est un entier égal à 0,
avec introduction éventuelle dans le milieu réactionnel, avant et/ou pendant et/ou après la mise en contact entre les constituant a), b) et c), d'au moins un agent de transfert de chaîne.

7. Procédé selon la revendication 6, **caractérisé en ce que** l'agent de transfert est mis en oeuvre et **en ce que** sa masse introduite dans le milieu réactionnel est comprise entre 1 500 ppm et 4 000 ppm par rapport au poids total des constituants a), b) et c).

8. Procédé selon l'une des revendications 6 ou 7, **caractérisé en ce que**, pour le monomère de formule (I), on a n = 0, AO désigne l'oxyde d'éthylène, et m est compris entre 20 et 40.

9. Procédé selon l'une des revendications 6 à 8, **caractérisé en ce que** le milieu réactionnel est constitué d'eau avec un autre solvant organique, préférentiellement uniquement constitué d'eau.

10. Procédé selon l'une des revendications 6 à 9, **caractérisé en ce que** l'agent de transfert de chaîne est choisi parmi N-dodécyl mercaptan, le N-décyl mercaptan, l'octyl mercaptan, le 1,8-dimercapto-3,6-dioxaoctane (DMDO, n° CAS : 14970-87-7), l'acide thiolactique.

11. Utilisation d'un polymère de type HASE selon l'une des revendications 3 à 5, comme agent épaississant dans une formulation aqueuse.

12. Formulation aqueuse contenant un polymère de type HASE selon l'une des revendications 3 à 5, **caractérisée en ce qu'**elle est une peinture aqueuse.

## Patentansprüche

1. Monomer der Formel (I) :
R-(AO)ₘ-(BO)ₙ-R'
wobei:
- m und n ganze Zahlen kleiner 150 sind, von denen mindestens eine nicht null ist,
- A und B für voneinander verschiedene Alkylgruppen mit 2 bis 4 Kohlenstoffatomen stehen, wobei die Gruppe AO vorzugsweise für Ethylenoxid steht und die Gruppe BO vorzugsweise für Propylenoxid steht,
- R für eine polymerisierbare ungesättigte Funktion, vorzugsweise Methacrylat, steht,
- wobei R' **dadurch gekennzeichnet ist, dass** es aus mindestens einer Gruppe der Formel (II) besteht:
CH₃-(CH₂)ₚ-CH(CH₂)ᵣCH₃-(CH₂)_{q}-
wobei p und q für ganze Zahlen stehen, von denen mindestens eine nicht null ist, wobei 5 < p+ q < 13, und r für eine ganze Zahl mit einem Wert von 0 steht.

2. Monomer nach Anspruch 1, **dadurch gekennzeichnet, dass** n = 0, AO für Ethylenoxid steht und m zwischen 20 und 40 liegt.

3. Polymer vom HASE-Typ, bestehend aus:
a) (Meth)acrylsäure,
b) mindestens einem (Meth)acrylsäureester,
c) mindestens einem Monomer der Formel (I),
R-(AO)ₘ-(BO)ₙ-R'
wobei:
- m und n ganze Zahlen kleiner 150 sind, von denen mindestens eine nicht null ist,
- A und B für voneinander verschiedene Alkylgruppen mit 2 bis 4 Kohlenstoffatomen stehen, wobei die Gruppe AO vorzugsweise für Ethylenoxid steht und die Gruppe BO vorzugsweise für Propylenoxid steht,
- R für eine polymerisierbare ungesättigte Funktion, vorzugsweise Methacrylat, steht,
- wobei R' **dadurch gekennzeichnet ist, dass** es aus mindestens einer Gruppe der Formel (II) besteht:
CH₃-(CH₂)ₚ-CH(CH₂)ᵣCH₃-(CH₂)_{q}-
wobei p und q für ganze Zahlen stehen, von denen mindestens eine nicht null ist, wobei 5 < p+ q < 13, und r für eine ganze Zahl mit einem Wert von 0 steht.

4. Polymer nach Anspruch 3, **dadurch gekennzeichnet, dass** es, ausgedrückt in Gew.-% jedes seiner Monomere, aus:
a) 20 % bis 50 %, vorzugsweise 35 % bis 45 %, (Meth)acrylsäure,
b) 40 % bis 70 %, vorzugsweise 45 % bis 55 %, mindestens eines (Meth)acrylsäureesters,
c) 2 % bis 20 %, vorzugsweise 3 % bis 15 %, mindestens eines Monomers der Formel (I),
besteht.

5. Polymer nach einem der Ansprüche 3 oder 4, **dadurch gekennzeichnet, dass** für das Monomer der Formel (I) n = 0, AO für Ethylenoxid steht und m zwischen 20 und 40 liegt.

6. Verfahren zur Herstellung eines Polymers vom HASE-Typ durch Inberührungbringen von:
a) (Meth)acrylsäure,
b) mindestens einem (Meth)acrylsäureester,
c) mindestens einem Monomer der Formel (I),
R-(AO)ₘ-(BO)ₙ-R'
wobei:
- m und n ganze Zahlen kleiner 150 sind, von denen mindestens eine nicht null ist,
- A und B für voneinander verschiedene Alkylgruppen mit 2 bis 4 Kohlenstoffatomen stehen, wobei die Gruppe AO vorzugsweise für Ethylenoxid steht und die Gruppe BO vorzugsweise für Propylenoxid steht,
- R für eine polymerisierbare ungesättigte Funktion, vorzugsweise Methacrylat, steht,
- wobei R' **dadurch gekennzeichnet ist, dass** es aus mindestens einer Gruppe der Formel (II) besteht:
CH₃-(CH₂)ₚ-CH(CH₂)ᵣCH₃-(CH₂)_{q}- (II)
wobei p und q für ganze Zahlen stehen, von denen mindestens eine nicht null ist, wobei 5<p+q<13, und r für eine ganze Zahl mit einem Wert von 0 steht,
in einem Reaktionsmedium, gegebenenfalls mit Einbringen mindestens eines Kettenübertragungsmittels in das Reaktionsmedium vor und/oder während und/oder nach dem Inberührungbringen der Bestandteile a), b) et c).

7. Verfahren nach Anspruch 6, **dadurch gekennzeichnet, dass** das Kettenübertragungsmittel so verwendet wird, dass dessen in das Reaktionsmedium eingetragen Masse zwischen 1500 ppm und 4000 ppm, bezogen auf das Gesamtgewicht der Bestandteile a), b) et c), liegt.

8. Verfahren nach einem der Ansprüche 6 oder 7, **dadurch gekennzeichnet, dass** für das Monomer der Formel (I) n = 0, AO für Ethylenoxid steht und m zwischen 20 und 40 liegt.

9. Verfahren nach einem der Ansprüche 6 bis 8, **dadurch gekennzeichnet, dass** das Reaktionsmedium aus Wasser mit einem anderen organischen Lösungsmittel, vorzugsweise einzig und allein aus Wasser, besteht.

10. Verfahren nach einem der Ansprüche 6 bis 9, **dadurch gekennzeichnet, dass** das Kettenübertragungsmittel aus N-Dodecylmercaptan, N-Decylmercaptan, Octylmercaptan, 1,8-Dimercapto-3,6-dioxaoctan (DMDO, CAS-Nr.: 14970-87-7) und Thiomilchsäure ausgewählt wird.

11. Verwendung eines Polymers vom HASE-Typ nach einem der Ansprüche 3 bis 5 als Verdickungsmittel in einer wässrigen Formulierung.

12. Wässrige Formulierung, die ein Polymer vom HASE-Typ nach einem der Ansprüche 3 bis 5 enthält, **dadurch gekennzeichnet, dass** es sich um ein wässriges Anstrichmittel handelt.

## Claims

1. A monomer of formula (I):
R-(EO)ₘ-(PO)ₙ-R'
where:
- m and n are integers of less than 150, at least one of which is non-zero,
- A and B designate alkyl groups different from one another, and having 2 to 4 carbon atoms, wherein group EO preferentially designates ethylene oxide, and group PO preferentially designates propylene oxide,
- R designates a polymerisable unsaturated group, preferentially methacrylate,
- R' is **characterised in that** it consists in at least one group of formula (II):
CH₃-(CH₂)ₚ-CH(CH₂)ᵣCH₃-(CH₂)_{q}-
where p and q designate integers at least one of which is non-zero, with 5 < p + q < 13, r is an integer equal to 0.

2. Monomer according to claim 1, **characterised in that** n = 0, EO designates the ethylene oxide, and m is comprised between 20 and 40.

3. A HASE-type polymer, consisting of:
a) (meth)acrylic acid,
b) at least one ester of (meth)acrylic acid,
c) at least one monomer of formula (I),
R-(EO)ₘ-(PO)ₙ-R'
where:
- m and n are integers of less than 150, at least one of which is non-zero,
- A and B designate alkyl groups different from one another, and having 2 to 4 carbon atoms, where group EO preferentially designates ethylene oxide, and group PO preferentially designates propylene oxide,
- R designates a polymerisable unsaturated group, preferentially methacrylate,
- where R' is **characterised in that** it consists in at least one group of formula (II):
CH₃-(CH₂)ₚ-CH(CH₂)ᵣCH₃-(CH₂)_{q}-
where p and q designate integers at least one of which is non-zero, with 5 < p + q < 13, r is an integer equal to 0.

4. Polymer according to claim 3, **characterised in that** it consists, expressed as a % by weight of each of its monomers:
a) From 20% to 50%, preferentially from 35% to 45%, of (meth)acrylic acid,
b) From 40% to 70%, preferentially from 45% to 55%, of at least one ester of (meth)acrylic acid,
c) From 2% to 20%, preferentially from 3% to 15%, of at least one monomer of formula (I).

5. Polymer according to one of claims 3 or 4, **characterised in that**, for the monomer of formula (I), n = 0, EO designates the ethylene oxide, and m is comprised between 20 and 40.

6. A method of manufacture of a HASE-type polymer, by contact in a reactive medium between:
a) (meth)acrylic acid,
b) at least one ester of (meth)acrylic acid,
c) at least one monomer of formula (I),
R-(EO)ₘ-(PO)ₙ-R'
where:
- m and n are integers of less than 150, at least one of which is non-zero,
- A and B designate alkyl groups different from one another, and having 2 to 4 carbon atoms, where group EO preferentially designates ethylene oxide, and group PO preferentially designates propylene oxide,
- R designates a polymerisable unsaturated group, preferentially methacrylate,
- R' is **characterised in that** it consists in at least one group of formula (II):
CH₃-(CH₂)ₚ-CH(CH₂)ᵣCH₃-(CH₂)_{q}- (II)
where p and q designate integers at least one of which is non-zero, with 5 < p+ q < 13, r is an integer equal to 0,
with possible introduction in the reactive medium, before and/or during and/or after the contact between the constituents a), b) and c), of at least one chain transfer agent.

7. A method according to claim 6, **characterised in that** the transfer agent is implemented and **in that** its mass introduced into the reactive medium is comprised between 1,500 ppm and 4,000 ppm based on the total weight of constituents a), b) and c).

8. A method according to one of claims 6 or 7, **characterised in that**, for the monomer of formula (I), n = 0, EO designates the ethylene oxide, and m is comprised between 20 and 40.

9. A method according to one of claims 6 to 8, **characterised in that** the reactive medium consists in water with another organic solvent, preferentially consisting only in water.

10. A method according to one of claims 6 to 9, **characterised in that** the chain transfer agent is chosen from among N-dodecyl mercaptan, N-decyl mercaptan, octyl mercaptan, 1,8-dimercapto-3,6-dioxaoctane (DMDO, n° CAS: 14970-87-7), thiolactic acid.

11. Use of a HASE-type polymer according to one of claims 3 to 5, as a thickening agent in an aqueous formulation.

12. An aqueous formulation containing a HASE-type polymer according to one of claims 3 to 5, **characterised in that** it is a water-based paint.
